# EUROPÄISCHE PATENTSCHRIFT

(11) **EP 0 315 656 B1**
(45) Veröffentlichungstag und Bekanntmachung des Hinweises auf die Patenterteilung: **07.07.1993**
(21) Anmeldenummer: 88904197.6
(22) Anmeldetag: 18.05.1988
(51) Int. Cl.: A61M 5/14

(54) **INFUSIONSGERÄT**
INFUSION APPARATUS
APPAREIL POUR FAIRE DES PERFUSIONS

(30) Priorität: 18.05.1987 CH 1905/87; 27.07.1987 CH 2851/87
(43) Veröffentlichungstag der Anmeldung: 17.05.1989
(73) Patentinhaber: DISETRONIC AG, 3400 Burgdorf (CH)
(72) Erfinder: MICHEL, Peter, CH-3400 Burgdorf (CH); MÜLLER, Hans, CH-3654 Gunten (CH)
(74) Vertreter: Lusuardi, Werther Giovanni, Dr.
(86) Internationale Anmeldenummer: CH8800092
(87) Internationale Veröffentlichungsnummer: WO8809187

(56) Entgegenhaltungen:
- DE-A- 3 446 909
- DE-A- 3 621 846
- FR-A- 2 195 461
- GB-A- 2 063 371

## Beschreibung

Die Erfindung bezieht sich auf ein Infusionsgerät gemäss dem Oberbegriff des Anspruchs 1.

Aus der WO88/00065 ist ein gattungsgemässes Infusionsgerät bekannt, mit welchem eine Flüssigkeit mittels eines Gaserzeugers durch eine lyophobe Kapillare als Druckschleuse förderbar ist. Nachteilig bei diesem bekannten Infusiongerät ist die Gefahr eines Druckstaus, was für Geräte, welche am Patienten getragen werden äusserst gefährlich sein kann. Aus der FR-A 2.195.461 ist ein weiteres Infusionsgerät bekannt, bei dem das zur Abgabe der Infusionsflüssigkeit benötigte Druckgas entweder durch Osmose, Elektroosmose oder durch elektrolytische Gaserzeugung mittels einer stromliefernden Batterie erzeugt wird. Nachteilig bei diesem Infusiongerät ist dessen komplizierter Aufbau, die geringe Betriebssicherheit und dessen Abhängigkeit von einer externen Stromquelle.

Hier will die Erfindung Abhilfe schaffen. Die Erfindung, wie sie in den Ansprüchen gekennzeichnet ist, löst die Aufgabe, ein Infusionsgerät zu schaffen, das preiswert, klein, leicht, zuverlässig und funktionssicher, einfach zu handhaben, in jeder Lage betriebsfähig, für ambulante Verwendung geeignet ist und auch am Körper des Patienten ohne Gefahr getragen werden kann.

Im folgenden wird die Erfindung anhand von lediglich einen Ausführungsweg darstellenden Zeichnungen näher erläutert. Es zeigt:
- Fig. 1: einen Achsenlängsschnitt durch ein erstes Ausführungsbeispiel des Infusionsgerätes mit einer zugehörigen Anschlusseinheit, in aufgegliederter Darstellung,
- Fig. 2: eine schematische Darstellung möglicher Ausschüttungsraten,
- Fig. 3: einen Achsenlängsschnitt durch ein Variante des Ausführungsbeispiels nach Fig. 1,
- Fig. 4: eine aufgegliederte Darstellung eines bevorzugten, zweiten Ausführungsbeispiels des Infusionsgerätes,
- Fig. 5: eine Darstellung des Ausführungsbeispiels von Fig. 4 in betriebsbereitem Zustand, teilweise im Schnitt, und
- Fig. 6: einen Achsenlängsschnitt durch den in die Ampulle eingesetzten Einsatzkopf des betriebsbereiten Infusionsgerätes gemäss Fig. 5, in grösserem Massstab.

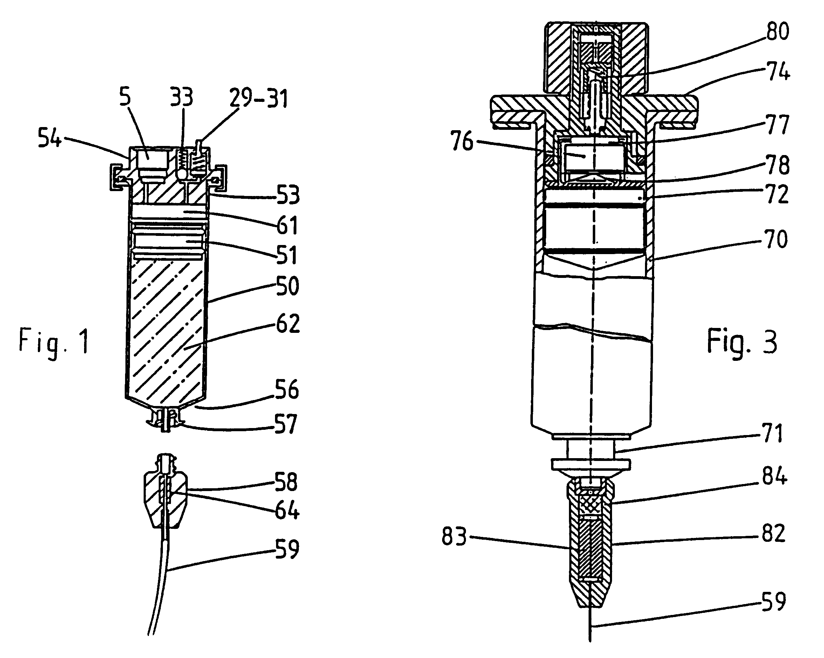

Das in Fig. 1 dargestellte Infusionsgerät besteht in seinem grundsätzlichen Aufbau aus einer die Infusionsflüssigkeit enthaltenden zylinderförmigen Ampulle 50 aus durchsichtigem Material, so dass deren Füllzustand überwacht und der Fortgang der Infusion verfolgt werden kann. In der Ampulle 50 ist ein Kolben 51 leicht verschiebbar gelagert. Die Ampulle 50 ist an einem Ende 53 durch einen als Deckel für das hintere Ampullenende ausgebildeten, dicht an diesem befestigbaren Einsatzkopf 54 geschlossen. Das andere Ende 56 der Ampulle 50 ist konisch verjüngt und mit einem Ausgangsansatz 57 versehen, der zum Anschliessen eines Anschlussstückes 58 an einem Ende eines Schlauchs 59 ausgebildet ist, der als Infusionskatheter zu verwenden oder am anderen (nicht dargestellten) Ende mit einer Infusionsnadel versehen ist. Die Ampulle 50 mit der Verjüngung 56 und dem Ausgangsansatz 57 und der Kolben 51 (ohne Kolbenstange) können ein Teil einer für medizinische Zwecke handelsüblichen Kolbenspritze sein.

Der Einsatzkopf 54 ist lösbar mit der Ampulle 50 verbunden und enthält einen Gaserzeuger 5,33 zur Erzeugung eines konstanten Überdruckes im hinteren Teil des Zylinderraumes. Der Gaserzeuger 5,33 besteht aus einer galvanischen Zelle 5 und einem bei dem gewünschten Überdruck ansprechenden Überdruckventil 33. Die galvanische Zelle 5 ist beispielsweise von der aus der DE-OS 35.32.335 beschriebenen Art. Die galvanische Zelle 5 ist durch einen in Fig. 1 nicht dargestellten Widerstand belastet und liefert eine durch die Belastung bestimmte, konstante Menge Wasserstoff oder Sauerstoff je Zeiteinheit.

Die das Gas erzeugende galvanische Zelle 5 ist in dem als Deckel für das hintere Ampullenende ausgebildeten, dicht an diesem befestigbaren Einsatzkopf 54 angeordnet. Ebenfalls im Einsatzkopf 54 angeordnet sind ein Druckanzeiger 29 - 31 und ein Überdruckventil 33, welches den Gasdruck im hinteren Teil des Zylinderraumes konstant hält. Der Druckanzeiger 29 - 31 besteht aus einem durch eine Feder 29 belasteten und mit einer Skala versehenen Stift 30 verbundenen Kolben 31. Der durch die Druckregelung verursachte Gasverlust ist tragbar, weil handelsübliche, galvanische Zellen dieser Art beim Zusammenwirken mit dem Überdruckventil 33 eine mindestens für eine Infusion ausreichende Gasmenge liefern.

Wie ersichtlich, ist der Ampullenraum durch den Kolben 51 in zwei Teilräume getrennt, deren einer 61 mit der galvanischen Zelle 5 zur Druckgaserzeugung verbunden ist, und deren anderer 62 zur Aufnahme der Infusionsflüssigkeit dient und mit dem Schlauch 59 verbindbar ist. Dabei überträgt der Kolben 51 den Gasdruck auf die Infusionsflüssigkeit, ohne dass das Gas mit dieser in Berührung kommt.

Der Kolben 51 kann an seiner dem Einsatzkopf 54 zugewandten Seite zum Angreifen eines Hilfsmittels ausgebildet sein, mit dem bei abgenommenem Einsatzkopf 54 in der in Fig. 1 dargestellten Lage des Gerätes zum Füllen desselben nach oben gezogen werden kann, wobei die Infusionsflüssigkeit am Ausgangsstutzten 57 angesaugt wird. Dazu kann der Kolben 51 mit einer Vertiefung versehen sein, die durch einen Steg überbrückt ist, an dem eine Pinzette oder ein Haken angreifen kann, oder die Vertiefung kann zum Einführen eines durch eine Drehung zugfest mit dem Kolben 51 verbindbaren Schlüssels geformt oder mit einem Gewindeloch versehen sein, in das ein Gewindestift einer Handhabe zum Zurückziehen des Kolbens 51 geschraubt werden kann. Für diese Art des Füllens oder Nachfüllens des Gerätes ist die Verschiebung des Kolbens 51 zweckmässig durch einen (nicht dargestellten) Anschlag am Ampullenende 53 begrenzt.

Um Luft aus dem Raum 62 (und gegebenenfalls dem angeschlossenen Schlauch 59) auszutreiben, kann - entsprechend dem üblichen Vorgehen bei der Verwendung von Kolbenspritzen - das Gerät mit dem Ausgangsstutzen 57 nach oben gehalten und von Hand auf den Kolben 51 gedrückt werden. Dabei ist die konische Verjüngung am Ampullenende 56 wesentlich, weil sie Luftblasen zwangsläufig zum Ausgangsstutzen 57 führt.

Bei Nichtgebrauch des gefüllten oder teilweise gefüllten Gerätes kann der Ausgangsansatz 57 durch eine Kappe verschlossen werden, im dargestellten Beispiel einer Schraubverbindung des Anschlussstückes 58 mit dem Ausgangsansatz 57 durch eine (nicht dargestellte) Schraubkappe.

Das Anschlussstück 58 enthält eine Eng- oder Drosselstelle, die im dargestellten Beispiel durch eine, in dem aus Kunststoff bestehenden Anschlussstück 58 eingebettete Glaskapillare 64 gebildet ist und zusammen mit dem Gasdruck die Durchflussrate der Infusionsflüssigkeit bestimmt.

Mit dem Gerät kann, nach Aufbau des Gasdrucks, eine im wesentlichen konstante Ausschüttungsrate der Infusionsflüssigkeit appliziert werden. Es kann aber auch eine schrittweise ändernde Auschüttungsrate und zusätzlich jeweils ein Bolus appliziert werden, indem der Gasdruck im Raum 61 und/oder der Strömungswiderstand der als Eng- oder Drosselstelle wirkenden Glaskapillare 64 geändert wird. Zur Änderung des Gasdrucks kann der die Anode und Kathode der galvanischen Zelle 5 verbindende Belastungswiderstand geändert werden, indem, z.B. ein als Belastungswiderstand wirkendes Halbleiterelement, zweckmässig ein Transistor, angesteuert wird. Auch eine Programmsteuerung für den Druck kann vorgesehen werden. Wenn der Druckerzeuger eine galvanische Zelle mit einem Transistor als Belastungswiderstand ist, kann an Stelle der Druckregelung mit dem Überdruckventil 33 eine elektrische Regelung verwendet werden, bei der ein z.B. piezoelektrischer Drucksensor den Transistor steuert.
Statt nach dem Programm kann der Strömungswiderstand der als Eng- oder Drosselstelle wirkenden Glaskapillare 64 und/oder der Druck im Raum 61 auch von einer den Patienten überwachenden Einrichtung in Abhängigkeit vom jeweiligen Zustand des Patienten (z.B. Puls, Blutzucker) gesteuert werden.

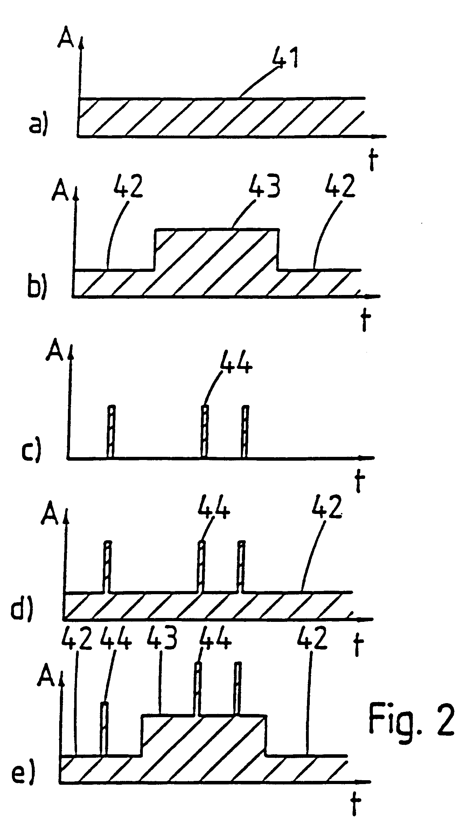

In Fig. 2 sind einige dieser Möglichkeiten lediglich schematisch dargestellt, wobei in der Realität die Ausschüttung bei Beginn der Gaserzeugung nicht sofort in Gang kommt, sondern mit Verzögerung auftritt und erst nach einer halben bis einer Stunde die gewünschte Ausschüttungsrate erreicht wird, und die Ausschüttungsrate bei Änderung des Gasdrucks nicht abrupt sondern allmählich ändert. In Fig. 2a ist eine konstante Ausschüttungsrate (A) 41 ohne zusätzlichen Bolus, in Fig. 2b eine stufenweise ändernde Ausschüttungsrate 42,43,42, in Fig. 2d eine konstante Auschüttungsrate 42 mit einem unregelmässig wiederholten Bolus 44 dargestellt. Fig. 2e zeigt eine Applikation, bei welcher der stufenweise ändernden Ausschüttungsrate 42,43,42 der Bolus 44 wiederholt überlagert ist. Nach Fig. 2c wird nur der Bolus 44 in zeitlicher Wiederholung appliziert. Zu diesem Zweck ist in Reihe mit der als Eng- oder Drosselstelle wirkenden Glaskapillare 64 ein (nicht dargestelltes) Ventil anzuordnen oder die Glaskapillare 64 durch ein Ventil zu ersetzen, das für die Anwendungen nach Fig. 2a,2b,2d und 2e zu öffnen, für die Anwendung nach Fig. 2c jedoch zu schliessen wäre.

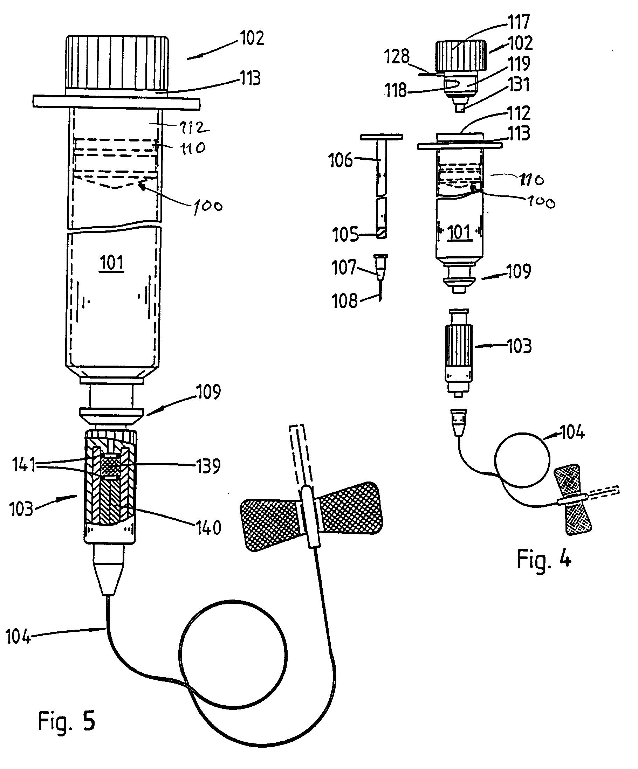

Bei einer (in Fig. 1 nicht dargestellten) Variante des Anschlussstücks 58 ist die Glaskapillare 64 durch einen Nebenschluss (Bypass) mit einem Ventil überbrückt, das die oben erläuterte Ausschüttungsmöglichkeit (Bolus) bietet. Ein entsprechender Bypass kann sinngemäss auch bei den nachfolgenden Ausführungsbeispielen von Fig. 3 und 4 vorgesehen sein.

Bei dem Gerät nach Fig. 3 sind der Zylinder 70 mit dem Ausgangsstutzen 71 und der Kolben 72 Teile einer handelsüblichen Kolben- oder Ampullenspritze, wobei der Kolben 72 an seiner dem Ausgangsstutzen 71 abgewandten Seite mit einem (nicht dargestellten) Gewindeloch versehen ist, so dass er bei abgenommenen Zylinderdeckel 74 mittels einer mit einem Gewindebolzen ausgerüsteten Handhabe zum Füllen des Zylinders 70 mit Infusionsflüssigkeit zurückgezogen werden kann, wie vorher erwähnt. Der Deckel enthält in koaxialer Anordnung eine das Gas entwickelnde, galvanische Zelle 76, die als Knopfzelle gemäss DE-OS 35 32 335 ausgeführt und durch einen Widerstand 77 belastet ist, und ein Ueberdruckventil 80. Der Widerstand 77 ist mit einem Pol der Knopfzelle 76 direkt und mit dem anderen über eine Kontaktfeder 78 verbunden. Dabei ist zwischen der Knopfzelle 76 und der Kontaktfeder 78 ein (nicht dargestelltes) Isolierplättchen geschoben, das am Umfang des Deckels 74 vorsteht und dort erfasst und herausgezogen werden kann, um den Stromkreis zu schliessen und die Zelle 76 zu aktivieren, bevor der Deckel 74 am Zylinder angebracht und das Gerät in Gebrauch genommen wird. Für denselben Zweck kann auch ein Kontakt vorgesehen werden, der selbsttätig schliesst, wenn der Deckel 74 am Zylinder 70 angebracht wird, und sich öffnet, wenn der Deckel wieder abgenommen wird.

Die Zelle 76 braucht nicht notwendig auswechselbar zu sein, sie kann mit dem Deckel 74 (und dem Ueberdruckventil 80) eine zum einmaligen Gebrauch bestimmte Einheit bilden. Solche Einheiten mit auf verschiedene Drücke eingestellten Ueberdruckventilen 80 können in unterschiedlichen Farben ausgeführt sein. Dies gilt auch für Anschlussstücke mit Kapillaren verschiedener Weite.

Der Zylinder 70 kann, wie vorher erwähnt, zum wiederholten Gebrauch jeweils gefüllt werden oder fabrikmässig gefüllt und zum einmaligen Gebrauch bestimmt sein, wobei der Ausgangsstutzen 71 mit einem abnehmbaren Verschluss auszurüsten oder von einer am Anschlussstück 82 angebrachten Hohlnadel zu durchstechen ist, wie vorher im Zusammenhang mit Fig. 1 erwähnt.

Auch kann der Zylinder 70 mit dem Kolben 72 und dem die Zelle 76 und das Ueberdruckventil 80 enthaltenden Deckel 74 eine fabrikmässig mit Infusionsflüssigkeit gefüllte Einheit zum einmaligen Gebrauch sein, wobei der Deckel 74 mit dem Zylinder 70 unlösbar verbunden sein kann und so auszuführen ist, dass das vorher erwähnte Isolierplättchen (abweichend von der Anordnung der Zelle 76 in Fig. 3) an dem aus dem Zylinder 70 herausragenden Teil des Deckels vorsteht, und zweckmässig gegen versehentliches Herausziehen gesichert ist.

Im Anschlussstück 82 ist vor der Kapillare 83 ein Filter 84 angeordnet, das eventuell in der Infusionsflüssigkeit vorhandene Teilchen zurückhält, welche die Kapillare 83 verstopfen könnten. Dieses Filter 84 und die anderen im Zusammenhang mit Fig. 3 erläuterten Einzelheiten sind auch bei den anderen Ausführungsarten des Infusionsgerätes sinngemäss anwendbar, und dies gilt auch umgekehrt.

Die in Fig. 4 - 6 dargestellte, bevorzugte Ausführungsform des Infusionsgeräts besteht aus einer mit einem Kolben 100 ausgerüsteten Ampulle 101, einem als Deckel für das hintere Ampullenende ausgebildeten Einsatzkopf 102 und einer aus einer Drosseleinrichtung 103 und einem Katheter 104 zusammengesetzten Anschlusseinheit. Zum Füllen der Ampulle 101 mit der Infusionsflüssigkeit ist eine am unteren Ende mit einem Gewinde 105 versehene Aufziehstange 106 und eine Anschlusseinheit 107 mit Kanüle 108 vorgesehen.

Die Ampulle 101 ist eine handelsübliche Spritzampulle, deren vorderes Ende mit einem Anschlussluer 109 versehen ist, und in der der ebenfalls handelsübliche Kolben 100 sitzt, der aus einem Gummistopfen 110 mit einem in diesen eingelassenen, ein Gewindesackloch aufweisenden Einsatz 111 besteht. Im hinteren Ampullenende sitzt eine Halterungshülse 112 für den Einsatzkopf 102, deren Aussenwandung dicht mit der Ampullenwandung verschweissten ist. Der hinterer Teil der Hülse hat eine glatte Innenwandung und einen auf dem Ampullenflansch anliegenden Kragen 113, der vordere ein Innengewinde 114 für den Einsatzkopf und einen ringförmig nach innen vorstehenden Anschlag 115 für den Kolben 100.

Der Einsatzkopf 102 hat einen mit einer Rändelung versehenen Kopfteil 117, dessen Durchmesser grösser als derjenige der Ampulle 101 ist, und einen von unten in den Kopfteil eingefügten, mit einem Aussengewinde 118 versehenen Schraubteil 119, in dem eine Vorschubeinheit für den Beginn des Kolbenvorschubs und eine galvanische Knopfzelle gemäss DE-OS 35 32 335 für den weiteren Kolbenvorschub angeordnet sind.

Zur Regelung des von der Knopfzelle 120 erzeugten Gasdrucks ist im Kopfteil 117 ein durch einen Verbindungskanal mit der Knopfzelle kommunizierendes Ueberdruckventil 121 angeordnet, dessen Ausgang in einen Auslass am Umfang des Kopfteils 117 mündet. Der Auslass ist durch eine Berstscheibe 122 verschlossen, die beim Ansprechen des Ueberdruckventils 121 birst und damit dessen Funktionsfähigkeit anzeigt.

Die Polkontakte (Anode und Kathode) der Knopfzelle 120 sind über eine Kontaktzunge 123 und eine Kontaktfeder 124 durch einen an der Unterseite einer Leiterplatte 125 angeordneten Widerstand 126 miteinander verbunden. Wie in Fig. 4 dargestellt, ist durch einen Schlitz 127 des Einsatzkopfes 102 ein ein Sicherheitselement bildendes Isolierplättchen 128 zwischen den Polkontakt der Knopfzelle 120 und die Kontaktfeder 124 geschoben, das seitlich aus dem Einsatzkopf 102 soweit herausragt, dass es bequem mit der Hand ergriffen und der Einsatkopf erst nach Herausziehen des Plättchens 128 und damit Inbetriebsetzen der Knopfzelle 120 in das hintere Ampullenende eingeführt werden kann.

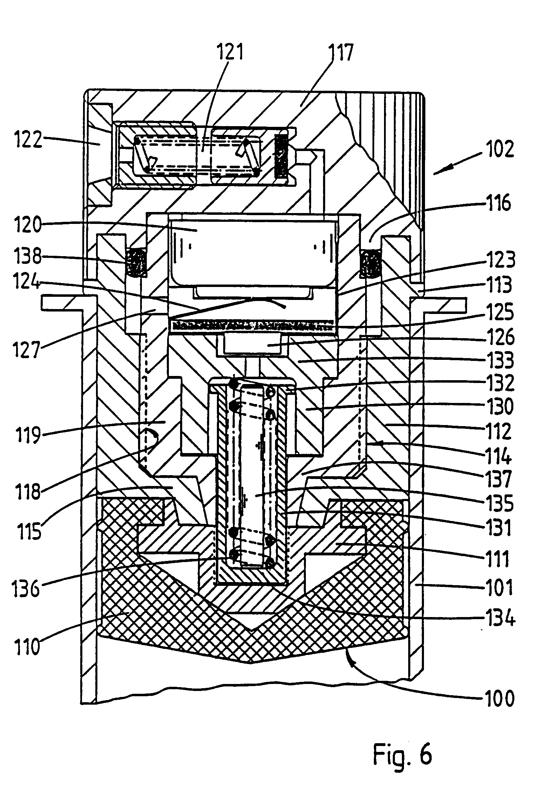

Die Vorschubeinheit umfasst einen topfförmigen, nach unten offenen und mit einem Gasdurchlass im Topfboden versehenen Teil 130, in den ein Stössel 131 hineinragt. Der Stössel 131 ist hohl, an seinem unteren Ende 134 geschlossen und an seinem oberen, offenen Ende mit einem der Topfinnenwandung angepassten Kragen 132 versehen. Zwischen den Topfboden 133 und den Stösselboden 134 ist eine um einen Bolzen 135 herumlaufende Druckfeder 136 eingespannt. (Der Bolzen 136 ist aus den weiter unten erläuterten Gründen der Luftraumverringerung vorgesehenen). Der Stössel 131 ist zwischen einer Stellung, in welcher der Kragen 132 an einem ringförmig nach innen ragenden Fortsatz 137 des Schraubteils 119 anschlägt, und einer Stellung, in welcher der Kragen 132 am Topfboden 133 anschlägt, hin und herverschiebbar im Fortsatz 137 gelagert. In der ersteren Stellung befindet sich der Stössel vor dem Einsetzen des Einsatzkopfes 102 (Fig. 4), in der letzteren unmittelbar nach dem Einschrauben des Einsatzkopfes 102 in die Halterungshülse 112 (Fig. 6).

Ein O-Ring 138 am Umfang des Einsatzkopfes 102 dichtet diesen beim Einsetzen an der Halterungshülse 112 ab, so dass das von der Zelle erzeugte Gas nicht austreten kann. Der O-Ring 138 liegt am oberen, gewindelosen Umfang des Schraubteils 119 und ist gegen ein nach oben Rutschen durch eine durch den Kopfteil 117 gebildete Stufe 116 gesichert.

Die Drosseleinrichtung 103 hat ein mit einem eingangsseitigen, dem Anschlussluer 109 angepassten, und einem ausgangsseitigen, einem Anschlussluer des Katheters 104 angepassten Anschlussstück versehenes, zylindrisches Gehäuse, in dem anschliessend an den Ausgangskanal des eingangsseitigen Anschlussstücks ein Filter 139 und ein Kapillarröhrchen 140 angeordnet sind. Das Filter 139 ist ein handelsübliches Medikamentenfilter, dessen Porenweite kleiner als der Innendurchmesser (40 Mikrometer) des Kapillarröhrchens 140 ist, damit dieses nicht verstopft werden kann. Das Filter 139 und das Kapillarröhrchen 140 sind mit an beiden Filterenden angeordneten O-Ringen 141 zwischen zwei Stirnwänden des zylindrischen Gehäuses zusammengepresst, damit die Infusionsflüssigkeit zuverlässig durch das Filter und das Kapillarröhrchen fliesst.

Zum Füllen der Ampulle 101 mit der Infusionsflüssigkeit wird die vorne mit dem Aussengewinde 105 versehene Aufziehstange 106 in das Gewindesackloch des Einsatzes 111 des Kolbens 100 geschraubt und die Anschlusseinheit 107 mit der Kanüle 108 auf den Anschlussluer 109 gesteckt, so dass die Ampulle 101 in der üblichen Weise gefüllt werden kann. Die Ampulle 101 muss vollständig gefüllt werden, was durch den Anschlag 115 der Halterungshülse 112 gewährleistet ist, an dem der Kolben 100 nach vollständiger Füllung anschlägt.

Nachdem die Ampulle 101 gefüllt ist, wird die Aufziehstange 106 aus dem Kolben 100 herausgeschraubt und die Anschlusseinheit 107 vom Luer 109 abgenommen. Die Drosseleinrichtung 103 wird ausgangsseitig an den Katheter 104 und eingangsseitig an den Luer 109 dicht angeschlossen. Danach wird das Isolierplättchen 128 aus dem Einsatzkopf 102 herausgezogen, und dieser von hinten in die Ampulle 101 eingesetzt und mit dem Aussengewinde 118 seines Schraubteils 119 in das Innengewinde 114 der Halterungshülse 112 geschraubt.

Beim Herausziehen des Isolierplättchens 128 ist zwar der Stromkreis zwischen Anode und Kathode der Zelle 120 über den Widerstand 126 geschlossen worden. Diese beginnt aber erst mit der Gasproduktion, wenn der Sauerstoff in der die Zelle umgebenden Luft aufgebraucht ist, und der Aufbau des Gasdrucks benötigt auch geraume Zeit, so dass der Gasarbeitsdruck erst nach einer halben bis einer Stunde aufgebaut ist. (Im Ausführungsbeispiel sind die Lufträume u. a. durch Einfügen des Bolzens 135 möglichst klein gehalten, trotzdem benötigt der Aufbau des Gasarbeitsdrucks die genannte Zeit). Obwohl der Gasarbeitsdruck nur langsam aufgebaut wird, beginnt aus den nachfolgend erläuterten Gründen unmittelbar nach dem Einschrauben des Einsatzkopfes 102 in die Ampulle der Kolbenvorschub und damit die Abgabe der Infusionsflüssigkeit. Das ist wesentlich, da für den Patienten eine sofortige Abgabe erforderlich sein kann oder zumindest erwünscht ist, und der Patient bei ausbleibender Abgabe zudem auf eine Funktionsstörung schliessen würde.

Beim Einschrauben des Schraubteils 119 wird der O-Ring 138 durch die Stufe 116 in die glatte zylindrische Wandung des oberen Teils der Halterungshülse 112 hineingepresst und gleitet an ihr nach unten, so dass die Luft zwischen dem O-Ring 138 und dem Kolben 100 komprimiert wird. Der Luftraum unter dem O-Ring 138 steht über die Gewinde 114, 118 und die Spalte zwischen der Halterungshülse 112, dem Schraubteil 119, dem Stössel 131, dem Vorschubteil 130 mit dem Durchgang im Topfboden 133 und der Leiterplatte mit dem Luftraum um die Zelle 120 sowie durch den Verbindungskanal mit dem Eingang des Ueberdruckventils 121 in Verbindung. Die Anordnung des O-Rings 138, d.h. die Weglänge, auf welcher der O-Ring 138 entlang der Halterungshülse bewegt wird, ist so im Verhältnis zu den Lufträumen gewählt, dass die komprimierte Luft einen Ueberdruck erreicht, bei dem das Ueberdruckventil 121 anspricht, so dass die Berstscheibe 122 bricht und damit das Funktionieren des Ueberdruckventils 121 angezeigt wird.

Beim Beginn des Einschrauben des Schraubteils 119 ist der Stössel 131 durch die Feder 136 nach unten gedrückt und sein Kragen 132 ist auf dem Fortsatz 137 abgestützt. Beim Weiterschrauben geht der (gewindelose) Stössel 131 in das Gewindesackloch des Einsatzes 111 des Kolbens 100. Da die Infusionsflüssigkeit nur sehr langsam durch das Filter 139 und das Kapillarröhrchen 140 austreten kann, wird der (zudem durch Haftreibung in der Ampulle 101 gehaltene) Kolben 100 durch den Flüssigkeitsdruck der Infusionsflüssigkeit gegen die Federkraft der Feder 136 gehalten und der Stössel 131 deshalb gegen die Federkraft nach oben in die in Fig. 6 gezeigte Lage bewegt.

Auf den Kolben 100 wirkt nun sowohl der Anfangsdruck der komprimierten Luft als auch - über den Stössel 131 - die Kraft der Feder 136, so dass der Kolben 100 unmittelbar nach dem Einschrauben des Einsatzkopfes 102 trotz noch nicht aufgebautem Gasdruck vorgeschoben und die Infusionsflüssigkeit durch die Drosseleinrichtung 103 und den Katheter 104 auszufliessen beginnt.

Die Feder 136 ist so bemessen, dass der vom Kolben 100 auf die Infusionsflüssigkeit bei Beginn des Vorschubs infolge der Federkraft ausgeübte Druck etwa gleich gross ist wie der vom Kolben 100 bei vollständig aufgebautem Gasdruck (bei unwirksamer Feder) auf die Infusionsflüssigkeit ausgeübte Druck, und dass der Kolben 100 während des Aufbaus des Gasdrucks durch die Zelle 120 mit stetig abnehmender Federkraft vorgeschoben wird, so dass sich durch die Ueberlagerung der abnehmenden Federkraft und des zunehmenden Gasdrucks ein annähernd konstanter Kolbenvorschub und eine dementsprechend konstante Ausschüttungsrate ergibt, die bei vollständig aufgebautem Gasdruck durch diesen allein weiter aufrechterhalten wird.

Unmittelbar nach dem Einschrauben des Einsatzkopfes 102 ist, da sowohl die Federkraft als auch die komprimierte Luft auf den Kolben 100 wirkt, der Kolbenvorschub kurzzeitig grösser als nach dem vollständigen Aufbau des Gasdrucks, was erwünscht ist, da vorerst die Einrichtung 103 und der Katheter 104 mit der Infusionsflüssigkeit gefüllt werden müssen. Bei vollständig aufgebautem Gasdruck schlägt der Kragen 132 des Stössels 131 am Fortsatz 137 an, so dass die Federkraft nicht mehr auf den sich durch den Gasdruck vom Stössel 131 wegbewegenden Kolben 100 wirkt. Der Widerstand 126 und das Ueberdruckventil 121 sorgen in der Folge dafür, dass der durch die Zelle erzeugte Gasdruck konstant bleibt. Der Widerstand 126 ist so bemessen, dass die Zelle genügend Gas liefert, um den für die gewünschte Ausschüttungsrate erforderlichen Gasarbeitsdruck aufrechtzuhalten; der Ueberdruck, bei dem das Ueberdruckventil 121 geöffnet wird, ist um eine Toleranz von ca. 0,2 bar höher eingestellt als der Gasarbeitsdruck.

Der Innendurchmesser des Kapillarröhrchens 140 ist so klein bemessen, vorzugsweise 40 Mikrometer, dass die durchströmende Infusionsflüssigkeit einen hohen (Reisungs-) Widerstand zu überwinden hat, der so hoch ist, dass die Reibung des Kolbens 100 und der Hautgegendruck des Patienten demgegenüber vernachlässigbar sind. Damit wird erreicht, dass sich Schwankungen der Kolbenreibung und des Hautgegendrucks praktisch nicht auf die Ausschüttungsrate (Infusionsrate) auswirken und mit einem hohen Gasarbeitsdruck gearbeitet werden kann, der eine zuverlässig konstante Vorschubgeschwindigkeit des Kolbens gewährleistet. Es hat sich gezeigt, dass die Ausschüttungsrate (Infusionsrate) etwa proportional zum Gasarbeitsdruck und indirekt proportional zur Viskosität und zur Länge des Kapillarröhrchens ist, jedoch mit der vierten Potenz vom Innendurchmesser des Kapillarröhrchens 140 abhängt, weshalb der Innendurchmesser sehr genau eingehalten werden muss, um die gewünschte Infusionsrate sicherzustellen.

Je nach Medikament und Indikation sind verschiedene Ausschüttungsraten von z. B. 0,5 - 4 ml/h und verschiedene Ampulleninhalte von z. B. 10 - 100 ml angezeigt. Die in Fig. 5 dargestellten Bauteile können als Bausatz für das Zusammensetzen von Infusionsgeräten mit unterschiedlicher Infusionsrate verwendet werden. Die Infusionsrate ist - bei gleich bemessener Drosseleinrichtung 103 (gleichem Innendurchmesser des Kapillarröhrchens) und gleicher Zelle 120 - nur durch den Widerstand 126 bestimmt, da der Gasfluss proportional zum Strom zunimmt. Der Bausatz besteht dementsprechend aus einem Satz von Ampullen 101 mit gleichem oder unterschiedlichem Volumen, und einem Satz von Einsatzköpfen 102, die für unterschiedliche Infusionsraten mit verschiedenen Widerständen 126 ausgerüstet und entsprechend gekennzeichnet sind. Dem jeweiligen Widerstand 126 bzw. dem diesem zugeordneten Gasarbeitsdruck sind die übrigen Ausrüstungselemente des Einsatzkopfes 102 angepasst, nämlich die Einstellung des Ueberdruckventils 121 und erforderlichenfalls auch die für den Anfangsdruck massgebende Feder 136 sowie die für die Luftkomprimierung massgebende Anordnung des O-Rings 138. Für eine Infusionsrate von 1 ml/h ist der Widerstand z. B. so zu bemessen, dass ein Gasarbeitsdruck von 1 bar aufrechterhalten wird, für eine Infusionsrate von 3 ml/h ein solcher von ca. 1,5 bar. Die Drosseleinrichtung 3 mit dem Katheter 104 und die Aufziehstange 106 sowie Anschlusseinheit 107 können für alle Infusionsgeräte gleich ausgeführt werden, wobei - wie erwähnt - der Innendurchmesser des Kapillarröhrchens 140 sehr genau eingehalten werden muss.

Für sehr hohe Infusionsraten können statt einer Zelle 120 auch mehrere Zellen 120 im Einsatzkopf 102 angeordnet sein.

## Patentansprüche

1. Infusionsgerät mit einem Raum (7; 62; 101) zur Aufnahme einer Infusionsflüssigkeit, der einen Ausgang (10; 57; 71; 109) für diese aufweist und durch eine bewegbare Wand (3; 51; 72; 100) veränderbar ist, die an der dem Raum abgewandten Seite durch den Druck eines Gases eines Gaserzeugers (5, 33; 76, 80; 120, 121) beaufschlagbar ist, dadurch gekennzeichnet, dass
A) der Gaserzeuger (5, 33; 76, 80; 120, 121) eine durch einen Widerstand (77; 126) belastete galvanische Zelle (5; 76; 120) ist, welche eine durch die Belastung bestimmte, konstante Menge Gas je Zeiteinheit erzeugt;
B) die bewegbare Wand von einem Kolben (51;72;100) einer die Infusionsflüssigkeit enthaltenden Ampulle (50;70;100) gebildet wird;
C) der Gaserzeuger (5, 33; 76, 80; 120, 121) in einem als Deckel für das hintere Ampullenende ausgebildeten, dicht an diesem befestigbaren Einsatzkopf (54;74;102) angeordnet ist; und
D) zur Regelung des vom Gaserzeuger (5, 33; 76, 80; 120, 121) erzeugten Gasdrucks ein Überdruckventil (33;80;121) oder ein den Widerstand (77; 126) steuernder Drucksensor vorgesehen ist.

2. Infusionsgerät nach Anspruch 1, dadurch gekennzeichnet, dass es als Kolbenspritze ausgebildet ist.

3. Infusionsgerät nach Anspruch 1 oder 2, dadurch gekennzeichnet, dass das Überdruckventil (33;80;121) im Einsatzkopf (54;74;102) angeordnet ist.

4. Infusionsgerät nach Anspruch 3, dadurch gekennzeichnet, dass der Ausgang des Überdruckventils (33;80;121) mit einem Auslass am aus der Ampulle (50;70;100) herausragenden Teil des Einsatzkopfes (54;74;102) in Verbindung steht.

5. Infusionsgerät nach Anspruch 4, dadurch gekennzeichnet, dass der Auslass durch eine Berstscheibe (122) verschlossen ist, die beim Ansprechen des Überdruckventils (33;80;121) birst und damit dessen Funktionsfähigkeit anzeigt.

6. Infusionsgerät nach einem der Ansprüche 3 - 5, dadurch gekennzeichnet, dass der Einsatzkopf (102) in das hintere Ampullenende hineinbewegbar ist und einen Dichtring (138) trägt, der beim Hineinbewegen in das hintere Ampullenende entlang einer zylindrischen Wandung (112) gleitet und die Luft zwischen dem Dichtring (138) und dem Kolben (100) komprimiert, so dass der Kolben (100) durch den Anfangsdruck der komprimierten Luft unmittelbar nach dem Einsetzen des Einsatzkopfes (102) vorgeschoben wird.

7. Infusionsgerät nach einem der Ansprüche 3 bis 6, dadurch gekennzeichnet, dass im Einsatzkopf (102) eine Feder (136) angeordnet ist, die bei am hinteren Ampullenende befestigen Einsatzkopf (102) auf den Kolben (100) wirkt, und deren Arbeitsweg so bemessen ist, dass sie den Kolben (100) während des Aufbaus des Gasdrucks durch den Gaserzeuger (120, 121) vorschiebt.

8. Infusionsgerät nach Anspruch 7, dadurch gekennzeichnet, dass die Feder (136) so bemessen ist, dass der vom Kolben (100) auf die Infusionsflüssigkeit bei Beginn des Vorschubs infolge der Federkraft und ggf. der komprimierten Luft ausgeübte Druck wenigstens annähernd so gross oder grösser ist wie der vom Kolben (100) nach vollständig aufgebautem Gasarbeitsdruck ohne Federkraft auf die Infusionsflüssigkeit ausgeübte Druck.

9. Infusionsgerät nach einem der Ansprüche 3 bis 8, dadurch gekennzeichnet, dass der Kolben (100) an seiner Rückseite (111) zum Angreifen eines Hilfsmittels (106) ausgebildet ist, mit dem der Kolben (100) bei noch nicht an der Ampulle (101) angebrachtem Einsatzkopf (102) zum Füllen der Ampulle (101) mit der Injektionsflüssigkeit zurückgezogen werden kann, und dass zwei wahlweise an das vordere Ampullenende (109) anschliessbare Anschlusseinheiten (107, 103/104) vorgesehen sind, deren eine für das Füllen der Ampulle (101) und deren andere für die Infusion ausgebildet ist.

10. Infusionsgerät nach einem der Ansprüche 1 bis 9, dadurch gekennzeichnet, dass der Gaserzeuger eine durch einen Widerstand (77; 126) belastete galvanische Zelle (5; 76; 120) ist.

11. Infusionsgerät nach Anspruch 10, dadurch gekennzeichnet dass die galvanische Zelle (5; 76; 120) durch einen verstellbaren Widerstand (77) oder durch mehrere, umschaltbare Widerstände belastet ist.

12. Infusionsgerät nach Anspruch 11, dadurch gekennzeichnet, dass der Widerstand steuerbar, z.B. ein Transistor ist, und dass ein Drucksensor vorgesehen ist, der den Widerstand zur Regelung des Ueberdruckes im zweiten Raum steuert.

13. Infusionsgerät nach einem der Ansprüche 10 bis 12, dadurch gekennzeichnet, dass in Reihe mit dem Belastungswiderstand (126) ein Kontakt (124) geschaltet ist, der im Ruhezustand des Gerätes offen und im Betriebszustand geschlossen oder zum Betrieb des Gerätes schliessbar ist.

14. Infusionsgerät nach den Ansprüchen 3 und 13, dadurch gekennzeichnet, dass der Kontakt durch eine Kontaktfeder (124) gebildet ist, die im Ruhezustand auf einem den Gegenkontakt isolierend abdeckenden Teil eines aus dem Einsatzkopf (102) herausragenden Sicherungselements (128) liegt, das zum Betrieb des Geräts aus dem Einsatzkopf (102) herausziehbar ist.

15. Infusionsgerät nach einem der Ansprüche 1 bis 14, dadurch gekennzeichnet, dass der Ausgang (10; 109) des Raums (7; 62, 101) mit einer vorzugsweise durch ein Kapillarrohr (140) gebildeten Eng- oder Drosselstelle (19; 64; 83; 140) für die zu applizierende Infusionsflüssigkeit kommuniziert.

16. Infusionsgerät nach Anspruch 15, gekennzeichnet durch ein zwischen dem Ausgang (10; 109) und der Eng- oder Drosselstelle (83; 140) angeordnetes Filter (84; 139), dessen Porenweite kleiner als die Weite der Eng- oder Drosselstelle (83; 140) ist.

17. Infusionsgerät nach den Ansprüchen 9, 15 und 16, dadurch gekennzeichnet, dass das Filter (84; 139) und die vorzugsweise durch ein Kapillarrohr (140) gebildete Eng- oder Drosselstelle (83; 140) in der Anschlusseinheit (103/104) für die Infusion angeordnet sind.

18. Bausatz für das Zusammensetzen von Infusionsgeräten nach einem der Ansprüche 1 bis 17 mit unterschiedlicher Infusionsrate, gekennzeichnet durch einen Satz von je mit einem die bewegbare Wand bildenden Kolben (100) ausgerüsteten Ampullen (101) zur Aufnahme der Infusionsflüssigkeit, und einen Satz von als Deckel für das hintere Ampullenende ausgebildeten, dicht an diesem befestigbaren Einsatzköpfen (102), deren jeder sämtliche zur Erzeugung und Regelung des Gasdrucks sowie ggf. des Anfangsdrucks bestimmten Ausrüstungselemente (120, 121, 131, 138) enthält, wobei die Ausrüstungselemente jedes Einsatzkopfes (102) so aneinander angepasst sind, dass der für eine bestimmte Infusionsrate erforderliche Gasdruck erzeugt und aufrechterhalten wird, und mit verschiedenen bzw. verschieden eingestellten Ausrüstungselementen versehene Einsatzköpfe (102) für verschiedene Infusionsraten vorgesehen sind.

## Claims

1. Infusion device, comprising a chamber (7; 62; 101) for accommodation of an infusion liquid, said chamber having an outlet (10; 57; 71; 109) for the liquid and being variable in size by means of a movable wall (3; 51; 72; 100) which is impingeable on the side opposite to said chamber with the pressure of a gas of a gas generator (5, 33; 76, 80; 120, 121), characterized in that
A) said gas generator (5, 33; 76, 80; 120, 121) is a galvanic cell (5; 76; 120) with a load resistor (77; 126) connected across said cell (5; 76; 120), which delivers a constant quantity of gas per unit of time determined by said load resistor (77; 126);
B) said movable wall (3; 51; 72; 100) is formed by a piston (51;72;100) of an ampoule (50;70;101) containing said infusion liquid;
C) said gas generator (5, 33; 76, 80; 120, 121) is positioned in an insert head (54;74;102) designed as a cover for the rear end of said ampoule (50;70;101) attachable in gas tight relation thereto; and
D) a pressure relief valve (33;80;121) or a pressure sensor controlling said load resistor (77; 126) is provided for regulating the gas pressure produced by said gas generator (5, 33; 76, 80; 120, 121).

2. Infusion device according to claim 1, characterized in that it is designed as a piston syringe.

3. Infusion device according to claim 1 or 2, characterized in that said pressure relief valve (33;80;121) is positioned in said insert head (54;74;102).

4. Infusion device according to claim 3, characterized in that the outlet of said pressure relief valve (33;80;121) is connected to an exit at the part of said insert head (54;74;102) protruding from said ampoule (50;70;101).

5. Infusion device according to claim 4, characterized in that said exit is sealed by a bursting disk (122) adapted to rupture when said pressure relief valve (33;80;121) operates thereby indicating its operativeness.

6. Infusion device according to one of the claims 3 - 5, characterized in that the insert head (54;74;102) is movable into the rear end of said ampoule (50;70;101) and bears a sealing ring (138) which upon moving into said rear end of said ampoule (50;70;101) is gliding along a cylindrical wall (112) and compresses the air between said sealing ring (138) and the piston (100) in such a way that the piston (100) is advanced by the initial pressure of the compressed air immediately after insertion of the insert head (54;74;102).

7. Infusion device according to one of the claims 3 - 6, characterized in that a spring (136) is positioned in the insert head (54;74;102) to act on said piston (100) when said insert head (54;74;102) is connected to said rear end of said ampoule (50;70;100), and said spring (136) having an operating path dimensioned so that said spring (136) advances the piston (100) during the buildup of gas pressure by said gas generator (5, 33; 76, 80; 120, 121).

8. Infusion device according to claim 7, characterized in that said spring (136) is dimensioned so that the pressure exerted by the piston (100) on the infusion fluid at the beginning of the advance on account of the spring force and optionally on account of the compressed air is at least approximately as high, or higher, as or than the pressure exerted by the piston (100) on the infusion fluid after the gas operating pressure has been completely built up, without a spring force.

9. Infusion device according to one of the claims 3 - 8, characterized in that said piston (100) is provided with means at its rear side (111) for application of auxiliary means (106) with which the piston (100) can be retracted for filling said ampoule (50;70;101) with said infusion liquid when said insert head (54;74;102) has not yet been attached to said ampoule (50;70;101) and that two connecting units (107, 103/104) are provided optionally connectable to the front end (109) of said ampoule (50;70;101), whereof one is for the filling of said ampoule (50;70;101) and the other for the infusion.

10. Infusion device according to one of the claims 1 - 9, characterized in that said gas generator (5, 33; 76, 80; 120, 121) is a galvanic cell (5; 76; 120) with a load resistor (77; 126).

11. Infusion device according to claim 10, characterized in that said galvanic cell (5; 76; 120) has a variable resistor (77) or several switchable resistors.

12. Infusion device according to claim 11, characterized in that said resistor is controllable, e.g. is a transistor, and that a pressure sensor is provided for controlling said resistor in order to regulate the gas overpressure.

13. Infusion device according to one of the claims 10 to 12, characterized in that a contact (124) is connected in series with said resistor (77;126) and said cell (5; 76; 120), means maintaining said contact in an open position in the inoperative condition of said device and closing said contact to complete a circuit between said cell and resistor in the operative condition of said device.

14. Infusion device according to claim 3 and 13, characterized in that said contact (124) is formed by a spring contact which in the inoperative condition of said device is resting on a safety element (128) protruding from said insert head (54;74;102) and insulating the counter contact, whereby said spring contact is extractable from said insert head (54;74;102) to operate said device.

15. Infusion device according to one of the claims 1 - 14, characterized in that the outlet (10;109) of said chamber (7; 62; 101) communicates with a liquid throttle member (19;64;83;140) for the infusion liquid to be administered to a patient, said throttle member (19;64;83;140) preferably being a capillary tube (14).

16. Infusion device according to claim 15, characterized in that a filter (84,139) is arranged between the outlet (10;109) and the throttle member (83;140), the pore width of this filter (84,139) being smaller than the width of the throttle member (83;140).

17. Infusion device according to claim 9, 15 and 16, characterized in that said filter (84,139) and said liquid throttle member (19;64;83;140), preferably in the form of a capillary tube (14), are positioned in the connecting unit (103/104) for the infusion.

18. Assembly kit for the assembly of infusion devices according to one of the claims 1 - 17 with different infusion rates, characterized by a kit of ampoules (101) for accommodation of the infusion liquid comprising a piston (100) forming the movable wall, a kit of insert heads (102) serving as cover for the rear end of said ampoule attachable in gas tight relation thereto, and of which each comprises all construction elements (120,121,131,138) for generating and controlling the gas pressure as well as optionally of the initial pressure, whereby the construction elements (120,121,131,138) of each insert head (102) are adapted in such a way to each other that the gas pressure necessary for a determined infusion rate is generated and maintained, and that insert heads (102) are provided with different, respectively differently set construction elements (120,121,131,138) for different infusion rates.

## Revendications

1. Dispositif de perfusion avec une enceinte (7; 62; 101) servant à reprendre un liquide à perfuser, présentant une sortie (10; 57; 71; 109) pour ce liquide et modifiable par une paroi mobile (3; 51; 72; 100) qui peut être sollicitée sur sa face opposée à l'enceinte par la pression du gaz d'un générateur de gaz (5, 33; 76, 80; 120, 121), caractérisé en ce que
A) le générateur de gaz (5, 33; 76, 80; 120, 121) est constitué par une cellule galvanique (5; 76; 120) chargée par l'intermédiaire d'une résistance (77; 126) qui crée un débit de gaz constant défini par la mise en charge;
B) la paroi mobile est formée par un piston (51; 72; 100) d'une ampoule (50; 70; 100) contenant le liquide à perfuser;
C) le générateur de gaz (5, 33; 76, 80; 120, 121) est installé hermétiquement sur une tête d'insertion (54; 74; 102) se présentant sous la forme d'un couvercle de l'extrémité arrière de l'ampoule et pouvant être fixé hermétiquement à celle-ci; et
D) une soupape de surpression (33; 80; 121) où une sonde de pression pilotant la résistance (77; 126) est prévue pour réguler la pression de gaz créée par le générateur de gaz (5, 33; 76, 80; 120, 121).

2. Dispositif de perfusion selon la revendication 1, caractérisé en ce qu'il se présente sous la forme d'une seringue à piston.

3. Dispositif de perfusion selon la revendication 1 ou 2, caractérisé en ce que la soupape de surpression (33; 80; 121) est installée dans la tête d'insertion (54; 74; 102).

4. Dispositif de perfusion selon la revendication 3, caractérisé en ce que la sortie de la soupape de surpression (5; 80; 121) communique avec une décharge située sur la partie, débordant de l'ampoule (50; 70; 100) de la tête d'insertion (54; 74; 102).

5. Dispositif de perfusion selon la revendication 4, caractérisé en ce que la décharge est refermée par un disque de rupture (122) qui lorsque la soupape de surpression (33; 80; 121) est sollicitée et indique ainsi qu'elle a fonctionné.

6. Dispositif de perfusion selon l'une des revendications 3-5, caractérisé en ce que la tête d'insertion (102) peut pénétrer dans l'extrémité arrière de l'ampoule et porte un anneau d'étanchéité (138) qui lors de la pénétration dans l'extrémité arrière de l'ampoule glisse le long d'une paroi cylindre (112) et comprime l'air situé entre l'anneau d'étanchéité (138) et le piston (100) de telle sorte que le piston (100) est poussé vers l'avant par la pression initiale de l'air comprimé, immédiatement après l'insertion de la tête d'insertion (102).

7. Dispositif de perfusion selon l'une des revendications 3 à 6, caractérisé en ce que dans la tête d'insertion (102) est installé un ressort (136) qui agit sur le piston (100) lorsque la tête d'insertion (102) est fixée contre l'extrémité arrière de l'ampoule, et donc le déplacement de travail est dimensionné de telle sorte qu'il pousse vers l'avant le piston (100) au cours de l'établissement de la pression de gaz par le générateur de gaz (120, 121).

8. Dispositif de perfusion selon la revendication 7, caractérisé en ce que le ressort 136 est dimensionné de telle sorte que la pression exercée par le piston (100) sur le liquide à perfuser au début de son déplacement vers l'avant suite à la force du ressort et éventuellement à l'air comprimé soit au moins approximativement aussi élevée ou supérieure à la pression exercée sans la force du ressort par le piston (100) sur le liquide à perfuser après que la pression de service du gaz se soit complètement établie.

9. Dispositif de perfusion selon l'une des revendications 3 à 8, caractérisé en ce que la face arrière (111) du piston (100) est configurée pour pouvoir accrocher un accessoire (106) grâce auquel le piston (100) peut être tiré vers l'arrière pour remplir l'ampoule (101) en liquide à injecter lorsque la tête d'insertion (102) n'est pas encore installée sur l'ampoule (101), et en ce que deux unités de raccordement (107, 103/104) sont prévues pour pouvoir se raccorder au choix à l'extrémité avant (109) de l'ampoule, l'une étant configurée pour le remplissage de l'ampoule (101) et l'autre l'étant pour la perfusion.

10. Dispositif de perfusion selon l'une des revendications 1 à 9, caractérisé en ce que le générateur de gaz est une cellule galvanique (5; 76; 120) mise en charge par l'intermédiaire d'une résistance (77; 126).

11. Dispositif de perfusion selon la revendication 10, caractérisé en ce que la cellule galvanique (5; 76; 120) est mise en charge par une résistance réglable (77) ou par plusieurs résistances commutables.

12. Dispositif de perfusion selon la revendication 11, caractérisé en ce que la résistance est modulable, et est par exemple un transistor, et en ce qu'une sonde de pression est prévue pour moduler la résistance en vue de la régulation de la surpression régnant dans la seconde enceinte.

13. Dispositif de perfusion selon l'une des revendications 10 à 12, caractérisé en ce qu'en série avec la résistance de charge (126) est branché un contact (124) ouvert lorsque le dispositif est à l'état de repos et fermé lorsqu'il est en position de fonctionnement, ou pouvant être refermé pour mettre le dispositif en fonctionnement.

14. Dispositif de perfusion selon les revendications 3 et 13, caractérisé en ce que le contact est réalisé par un ressort de contact (124) qui à l'état de repos repose sur une pièce, recouvrant et isolant le contact opposé, d'un élément de sécurité (128) débordant hors de la tête d'insertion (102), et qui peut être extrait hors de la tête d'insertion (102) pour mettre le dispositif en fonctionnement.

15. Dispositif de perfusion selon l'une des revendications 1 à 14, caractérisé en ce que la sortie (10; 109) de l'enceinte (7; 62, 101) communique avec un rétrécissement ou étranglement (19; 64; 83; 140) de l'écoulement du liquide de perfusion à administrer, formé de préférence par un tube capillaire (140).

16. Dispositif de perfusion selon la revendication 15, caractérisé par un filtre (84; 139) situé entre la sortie (10; 109) et le rétrécissement ou étranglement (83; 140), dont la largeur des pores est inférieure à la largeur du rétrécissement ou étranglement (83; 140).

17. Dispositif de perfusion selon les revendications 9, 15 et 16, caractérisé en ce que le filtre (84; 139) et le rétrécissement ou étranglement (83; 140) formé de préférence par un tube capillaire (140) sont disposés dans l'unité de raccordement (103/104) servant à la perfusion.

18. Trousse de montage pour l'assemblage de dispositifs de perfusion selon l'une des revendications 1 à 17, à différents débits de perfusion, caractérisée par un jeu d'ampoules (101) de reprise du liquide à perfuser et chacune équipée du piston (100) formant la paroi mobile, et par un jeu de têtes d'insertion (102) servant de couvercle pour l'extrémité arrière de l'ampoule et pouvant être fixées hermétiquement à ces dernières, et dont chacune contient l'ensemble des éléments d'équipement (120, 121, 131, 138) destinés à créer et réguler la pression de gaz ainsi qu'éventuellement la pression initiale, les éléments d'équipement de chaque tête d'insertion (102) étant adaptés les uns aux autres de manière à créer et maintenir la pression de gaz nécessaire pour un débit de perfusion donnée, des têtes d'insertion (102) dotées d'éléments d'équipement différemment réglés étant prévues pour différents débits de perfusion.
